# EUROPEAN PATENT APPLICATION

(11) **EP 4 467 180 A1**
(43) Date of publication of application: **27.11.2024**
(21) Application number: 23743371.9
(22) Date of filing: 23.01.2023
(51) Int. Cl.: A61M 21/02, F24F 11/62, F24F 11/64, F24F 11/65, F24F 11/74, F24F 11/79, F24F 11/80, F24F 110/00

(54) **ENVIRONMENTAL CONTROL DETAIL-DETERMINING DEVICE AND ENVIRONMENTAL CONTROL DETAIL DETERMINATION METHOD**

(30) Priority: 21.01.2022 JP 2022008305; 30.09.2022 JP 2022159006
(71) Applicant: DAIKIN INDUSTRIES, LTD., Osaka-shi, Osaka 530-0001 (JP); The University of Electro-Communications, Chofu-shi, Tokyo 182-8585 (JP)
(72) Inventor: SHIMADA, Miki, Osaka-shi, Osaka 530-0001 (JP); ASHIKAGA, Tomoyoshi, Osaka-shi, Osaka 530-0001 (JP); OHGA, Takahiro, Osaka-shi, Osaka 530-0001 (JP); TAKEUCHI, Toshifumi, Osaka-shi, Osaka 530-0001 (JP); TAKADAMA, Keiki, Chofu-shi, Tokyo 182-8585 (JP)
(74) Representative: Global IP Europe Patentanwaltskanzlei
(86) International application number: PCT/JP2023/001929
(87) International publication number: WO 2023/140379

(57) **Abstract**

There is a problem that a conventional technique is insufficient for providing a comfortable nap to a subject. An environmental control content determination device (1) includes an acquisition part (191) and a determination part (193). An acquisition part (191) acquires pre-nap information (D 1 1) and an expected nap time zone (D12) including an expected nap start time and an expected nap end time regarding a subject. The determination part (193) judges the wakefulness level of the subject in the expected nap time zone (D12) based on the pre-nap information (D11), and determines the content of environmental control for a target space in which the subject takes a nap.

## Description

### TECHNICAL FIELD

The present invention relates to an environmental control content determination device and an environmental control content determination method.

### BACKGROUND ART

In general, as disclosed in PTL 1 (Japanese Patent No. 6775949), there is a technique for performing environmental control on a target space, in which the subject takes a nap, to provide a comfortable nap to a subject.

### Summary of Invention

### <Technical Problem>

PTL 1 has a problem that it is not sufficient for providing a comfortable nap to the subj ect.

### <Solution to Problem>

A first aspect of the present invention provides an environmental control content determination device includes an acquisition part and a determination part. The acquisition part acquires pre-nap information and an expected nap time zone including an expected nap start time and an expected nap end time regarding a subject. The determination part judges a wakefulness level of the subject in the expected nap time zone based on the pre-nap information, and determines a content of environmental control for a target space in which the subject takes a nap.

The environmental control content determination device according to the first aspect judges the wakefulness level of the subject in the expected nap time zone based on the pre-nap information, and determines the content of environmental control for the target space in which the subject takes a nap. As a result, the environmental control content determination device can provide the subject with a more comfortable nap.

An environmental control content determination device of a second aspect is the environmental control content determination device of the first aspect, and further includes a prediction part. The prediction part predicts a cycle of sleepiness of the subject from the pre-nap information. The determination part determines the content of environmental control based on a temporal relationship between the expected nap time zone and the cycle of the sleepiness.

The cycle of sleepiness of the subject who takes a nap has not been conventionally taken into consideration, but the cycle of sleepiness has been now found to be an important factor for providing a comfortable nap to the subject. The environmental control content determination device of the second aspect determines the content of environmental control for the target space in which the subject takes a nap in consideration of the cycle of sleepiness of the subject who takes a nap. As a result, the environmental control content determination device can provide the subject with a more comfortable nap.

An environmental control content determination device of a third aspect is the environmental control content determination device of the first aspect or the second aspect, in which the pre-nap information includes at least first information including information related to sleep of the subject.

An environmental control content determination device of a fourth aspect is the environmental control content determination device of the third aspect, in which the information related to the sleep includes at least one of a wake-up time on the day of the nap, a bed time corresponding the wake-up time on the day of the nap, a sleep time corresponding the wake-up time on the day of the nap, an average wake-up time, an average bed time, an average sleep time, or an evaluation value of a daily sleeping state.

An environmental control content determination device of a fifth aspect is the environmental control content determination device of the third aspect or the fourth aspect, in which the first information further includes at least one of information related to meals, a subjective evaluation for sleepiness before the nap, a subjective fatigue evaluation before the nap, a subjective stress evaluation before the nap, a behavior expectation after the nap, or a behavior history on the day of the nap.

An environmental control content determination device of a sixth aspect is the environmental control content determination device of the fifth aspect, in which the information related to the meals includes at least one of: times when the subject has a breakfast, a lunch, a snack, and a dinner; or meal contents of the breakfast, the lunch, the snack, and the dinner of the subject.

An environmental control content determination device of a seventh aspect is the environmental control content determination device of any one of the third aspect to the sixth aspect, in which the pre-nap information further includes second information including at least one of a gender, an age, a generation, a clothing amount, or a thermal sensation of the subject.

An environmental control content determination device of an eighth aspect is the environmental control content determination device of any one of the third aspect to the seventh aspect, in which the pre-nap information further includes at least one of an eyelid opening/closing state, an eyelid motion, a respiration rate, a pulse, a blood pressure, an LF/HF, a brainwave, a body temperature, or an in-vivo blood glucose level of the subject.

An environmental control content determination device of a ninth aspect is the environmental control content determination device of any one of the first aspect to the eighth aspect, in which the determination part determines the content of environmental control by selecting from a plurality of control patterns.

The environmental control content determination device of the ninth aspect can simplify the environmental control by selecting the content of the environmental control from a plurality of control patterns.

An environmental control content determination device of a tenth aspect is the environmental control content determination device of the ninth aspect, in which the control pattern is expressed by a form of a transition in a value of a control parameter related to an environment of the target space.

An environmental control content determination device of an eleventh aspect is the environmental control content determination device of the tenth aspect, in which the control parameter includes at least one of: an air volume, a wind direction, a wind speed, or an operating mode of an air conditioner installed in the target space; an indoor temperature, an indoor humidity, an illumination, a sound, or a fragrance in the target space; or an inclination of bedding.

An environmental control content determination device of a twelfth aspect is the environmental control content determination device of the tenth aspect or the eleventh aspect, in which the control parameter is an indoor temperature of the target space. The control pattern includes a control to raise the indoor temperature before a sleep onset of the subject, to lower and maintain the indoor temperature after detection of the sleep onset, and to raise the indoor temperature at a predetermined time before the expected nap end time, and a control to raise the indoor temperature before a sleep onset of the subject and to lower and maintain the indoor temperature after detection of the sleep onset.

An environmental control content determination device of a thirteenth aspect is the environmental control content determination device of any one of the ninth aspect to the twelfth aspect, in which the determination part specifies, from the cycle of sleepiness, a first time zone in which the sleepiness is felt and sleep is easily taken for a short time and a second time zone in which the sleepiness is hardly felt and the sleep is hardly taken for a short time. The determination part selects the control pattern depending on whether the expected nap time zone corresponds to either of the first time zone and the second time zone.

With such a configuration, the environmental control content determination device of the thirteenth aspect can select the control pattern according to the cycle of sleepiness of the subj ect.

An environmental control content determination device of a fourteenth aspect is the environmental control content determination device of the thirteenth aspect, in which the determination part further selects the control pattern, based on whether a sleep time corresponding to a wake-up time on the day of the nap is shorter than a predetermined time with respect to an average sleep time of the subject, or whether an evaluation value of a daily sleeping state of the subject exceeds a threshold.

An environmental control content determination device of a fifteenth aspect is the environmental control content determination device of any one of the tenth aspect to the fourteenth aspect, in which the acquisition part further acquires intra-nap information regarding the subject during the nap of the subject. The acquisition part further acquires post-nap information regarding the subject after an end of the nap of the subject. The environmental control content determination device further includes an update part. The update part updates the form of the transition in the value of the control parameter. The update part updates the form of the transition in the value of the control parameter, based on the pre-nap information, the intra-nap information, or the post-nap information, after the end of the nap of the subject.

The environmental control content determination device of the fifteenth aspect can provide a more comfortable nap at the time of the next nap by updating the form of the transition in the value of the control parameter.

An environmental control content determination device of a sixteenth aspect is the environmental control content determination device of the fifteenth aspect, in which the intra-nap information includes at least one of: a sleep depth, a heartbeat, a body motion, a pulse, a respiration rate, an LF/HF, a blood pressure, a body temperature, a sleep onset latency, or a brainwave of the subject; an indoor temperature, an indoor humidity, an airflow, an illumination, a sound, or a fragrance in the target space; a temperature of bedding; an inclination of the bedding; or an outdoor temperature or an outdoor humidity outside the target space.

An environmental control content determination device of a seventeenth aspect is the environmental control content determination device of the fifteenth aspect or the sixteenth aspect, in which the post-nap information includes at least one of a subjective evaluation of the subject with respect to a level of satisfaction of the nap, a subjective evaluation of the subject with respect to a thermal sensation during the nap, or a subjective evaluation of the subject with respect to sleepiness after the nap.

An environmental control content determination device of an eighteenth aspect is the environmental control content determination device of any one of the fifteenth aspect to the seventeenth aspect, in which the form of the transition in the value of the control parameter for the subject is determined to be: a form of a transition in a value of the control parameter of a person who has the pre-nap information similar to that of the subject and is different from the subject; or a form of a transition in a value of the control parameter of a past person, who has determined by the update part that a sufficient nap has been taken.

An environmental control content determination device of a nineteenth aspect is the environmental control content determination device of any one of the fifteenth aspect to the eighteenth aspect, in which the pre-nap information includes a subjective evaluation for sleepiness before the nap. The intra-nap information includes at least one of a brainwave, a heartbeat, a pulse, an LF/HF, a sleep depth, a respiration rate, or a body temperature. The post-nap information includes at least one of a subjective evaluation of the subject with respect to a level of satisfaction of the nap, a subjective evaluation of the subject with respect to a thermal sensation during the nap, or a subjective evaluation of the subject with respect to sleepiness after the nap. The update part updates the form of the transition in the value of the control parameter when it is determined that the subject has not taken a sufficient nap or the subject has felt an unpleasant thermal sensation during the nap.

An environmental control content determination device of a twentieth aspect is the environmental control content determination device of the eighteenth aspect or the nineteenth aspect, in which the update part determines that the sufficient nap has not been taken when a nap time is shorter than a threshold, when a time of a predetermined sleep stage is shorter than a threshold, when a standard deviation of a heartbeat, a respiration rate, a pulse, or an LF/HF during sleep is larger than a threshold, or when a subjective evaluation for sleepiness after the nap is not better than a subjective evaluation for sleepiness before the nap.

An environmental control content determination method of a twenty-first aspect includes an acquisition step and a determination step. The acquisition step includes acquiring pre-nap information and an expected nap time zone including an expected nap start time and an expected nap end time regarding a subject. The determination step includes determining, based on the pre-nap information, a content of environmental control for a target space in which the subject takes a nap.

An environmental control content determination method of a twenty-second aspect is the environmental control content determination method of the twenty-first aspect, and further includes a prediction step. The prediction step includes predicting a cycle of sleepiness of the subject from the pre-nap information. The determination step including determining the content of environmental control based on a temporal relationship between the expected nap time zone and the cycle of the sleepiness.

### Brief Description of Drawings

[Fig. 1] Fig. 1 is a schematic configuration diagram of an environmental control content determination device and an air conditioner.
[Fig. 2] Fig. 2 is a functional block diagram of the environmental control content determination device.
[Fig. 3] Fig. 3 is a diagram showing a first control pattern.
[Fig. 4] Fig. 4 is a diagram showing a second control pattern.
[Fig. 5] Fig. 5 is a flowchart illustrating an example of processing of the environmental control content determination device.

### Description of Embodiments

### (1) Overall configuration

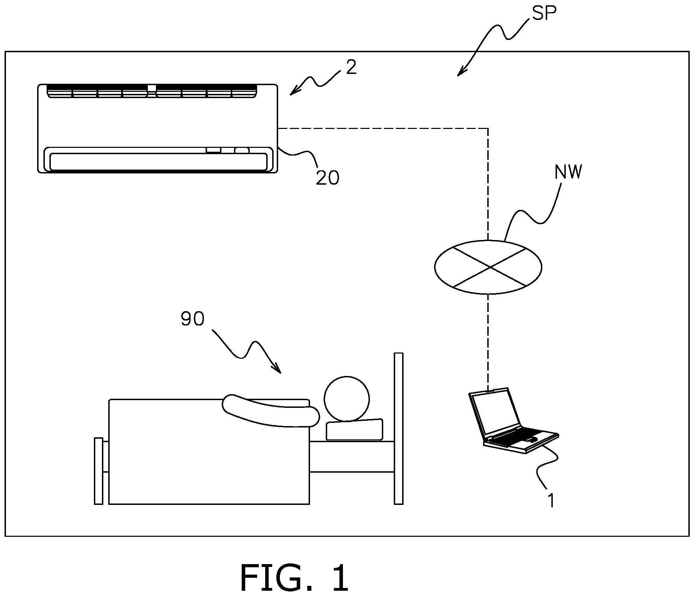

Fig. 1 is a schematic configuration diagram of an environmental control content determination device 1 and an air conditioner 2. As shown in Fig. 1, the environmental control content determination device 1 determines the content of environmental control for a target space SP in which a subject 90 takes a nap. In the present embodiment, the target space SP is assumed to be a private room for taking a nap. The environmental control content determination device 1 transmits the determined content of environmental control to the air conditioner 2 installed in the target space SP, and causes the air conditioner 2 to perform air conditioning (environmental control) of the target space SP.

The environmental control content determination device 1 and the air conditioner 2 are communicably connected to each other via a network NW. The network NW is, for example, a wireless LAN.

Further, various sensors (not shown) are installed inside the target space SP or outside the target space SP. These various sensors and the environmental control content determination device 1 are communicably connected to each other via the network NW.

### (2) Detailed configuration

### (2-1) Environmental control content determination device

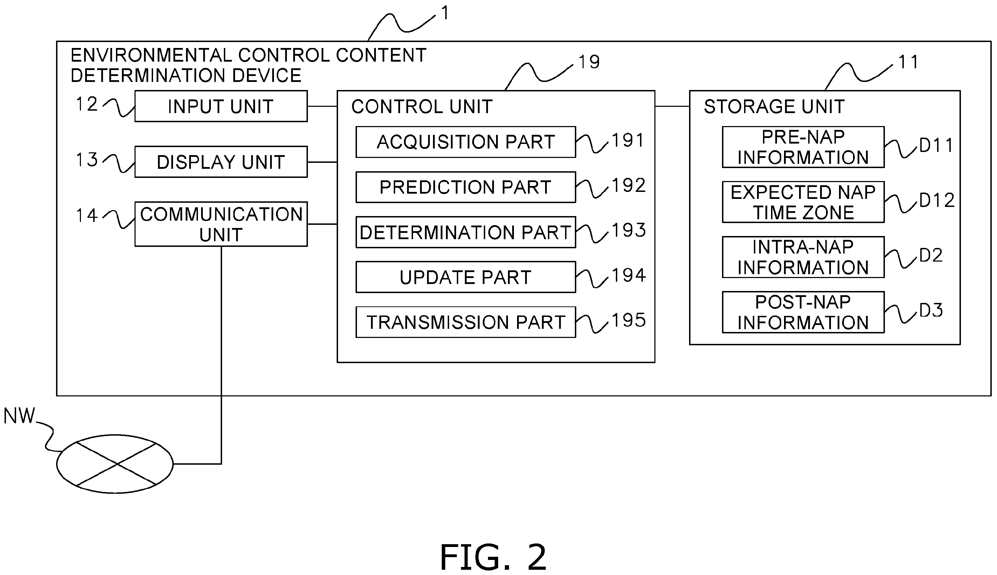

In the present embodiment, the environmental control content determination device 1 is a general computer. Fig. 2 is a functional block diagram of the environmental control content determination device 1. As shown in Fig. 2, the environmental control content determination device 1 mainly includes a storage unit 11, an input unit 12, a display unit 13, a communication unit 14, and a control unit 19.

### (2-1-1) Storage unit

The storage unit 11 is a storage device such as a RAM, a ROM, or an HDD. The storage unit 11 stores a program executed by the control unit 19, data necessary for execution of the program, and the like.

In the present embodiment, the storage unit 11 particularly stores pre-nap information D11, an expected nap time zone D12, intra-nap information D2, and post-nap information D3 which will be described below.

### (2-1-2) Input unit

The input unit 12 indicates a keyboard and a mouse. Various instructions or various kinds of information for the environmental control content determination device 1 can be input via the input unit 12.

The subject 90 inputs pre-nap information D11 and an expected nap time zone D12, which will be described below, via the input unit 12 before the start of the nap. Further, the subject 90 inputs post-nap information D3, which will be described below, via the input unit 12 after the end of the nap.

### (2-1-3) Display unit

The display unit 13 is a monitor. The display unit 13 can display an input screen for inputting various kinds of information to the environmental control content determination device 1, various kinds of information stored in the storage unit 11, and the like.

### (2-1-4) Communication unit

The communication unit 14 is a network interface device for communicating with the air conditioner 2 and the like via the network NW.

### (2-1-5) Control unit

The control unit 19 is a processor such as a CPU or a GPU. The control unit 19 reads and executes a program stored in the storage unit 11 to implement various functions of the environmental control content determination device 1. In addition, the control unit 19 can write an arithmetic result in the storage unit 11 or read out the information stored in the storage unit 11, according to a program.

As shown in Fig. 2, the control unit 19 includes, as functional blocks, an acquisition part 191, a prediction part 192, a determination part 193, an update part 194, and a transmission part 195.

### (2-1-5-1) Acquisition part

### (2-1-5-1-1) Pre-nap information and Expected nap time zone

The acquisition part 191 acquires, regarding the subject 90, the pre-nap information D11 and the expected nap time zone D12 including an expected nap start time and an expected nap end time.

The pre-nap information D11 includes at least first information including information related to sleep of the subject 90. The information related to the sleep includes at least one of a wake-up time on the day of the nap, a bed time corresponding the wake-up time on the day of the nap, a sleep time corresponding the wake-up time on the day of the nap, an average wake-up time, an average bed time, an average sleep time, or an evaluation value of a daily sleeping state. Here, it is generally assumed that the bed time corresponding to the wake-up time on the day of the nap is the night before the nap or a daybreak on the day of the nap. Further, the sleep time corresponding to the wake-up time on the day of the nap means a sleep time until the wake-up time on the day of the nap from the bed time corresponding to the wake-up time on the day of the nap. In addition, the evaluation value of the daily sleeping state is, for example, Epworth Sleepiness Scale (ESS or JESS) indicating subjective daytime sleepiness, 3 Dimensional Sleep Scale (3DSS) indicating sleep phase, quality, and quantity, Athens Insomnia Scale (AIS) indicating the degree of insomnia, or Pittsburgh Sleep Quality Index (PSQI) indicating an overall sleep quality or the like. In the present embodiment, as the information related to the sleep of the subject 90, the wake-up time on the day of the nap, the bed time corresponding to the wake-up time on the day of the nap, the sleep time corresponding to the wake-up time on the day of the nap, the average wake-up time, the average bed time, the average sleep time, and the evaluation value of the daily sleeping state are used.

The first information further includes at least one of information related to meals, a subjective evaluation for sleepiness before the nap, a subjective fatigue evaluation before the nap, a subjective stress evaluation before the nap, a behavior expectation after the nap, or a behavior history on the day of the nap. Here, the information related to meals includes at least one of the time when the subject 90 has a breakfast, a lunch, a snack, and a dinner or meal contents of the breakfast, the lunch, the snack, and the dinner of the subject 90. The meal contents include, for example, beverages containing caffeine and alcohol. In the present embodiment, as the first information other than the information related to the sleep of the subject 90, the subjective evaluation for sleepiness before the nap is used.

In addition, the pre-nap information D11 further includes second information including at least one of a gender, an age, a generation, a clothing amount, or a thermal sensation of the subject 90. In the present embodiment, the gender and the age of the subject 90 are used as the second information.

The expected nap time zone D12 is a time zone until the expected nap end time from the expected nap start time.

The acquisition part 191 acquires the pre-nap information D11 and the expected nap time zone D12 from the subject 90 via the input unit 12 before the start of the nap of the subject 90.

### (2-1-5-1-2) Intra-nap information

The acquisition part 191 acquires the intra-nap information D2 regarding the subject 90 during the nap of the subject 90. The intra-nap information D2 includes at least one of: a sleep depth, a heartbeat, a body motion, a pulse, a respiration rate, an LF/HF, a blood pressure, a body temperature, a sleep onset latency, or brainwave of the subject 90; an indoor temperature, an indoor humidity, an airflow, an illumination, a sound, or a fragrance in the target space SP; a temperature of bedding; an inclination of bedding; or an outdoor temperature or an outdoor humidity outside the target space SP. The heartbeat, the pulse, the respiration rate, the LF/HF, the blood pressure, the body temperature, and the brainwave of the subject 90 are acquired from, for example, a contact type vital sensor or a contactless type vital sensor installed in the target space SP. The sleep depth, the body motion, and the sleep onset latency of the subject 90 are acquired, from, for example, a sleep meter attached to the bedding. Here, the sleep depth is a time for each of six sleep stages including REM sleep, four stages of non-REM sleep, and awakening. The indoor temperature, the indoor humidity, the airflow, the illumination, the sound, and the fragrance of the target space SP are acquired from, for example, a temperature sensor, a humidity sensor, an airflow sensor, an illumination sensor, a noise sensor, and an odor sensor installed in the target space SP, respectively. The temperature of bedding and the inclination angle of bedding are acquired from, for example, a temperature sensor and an angle sensor attached to the bedding, respectively. The outdoor temperature and the outdoor humidity outside the target space SP are acquired from, for example, a temperature sensor and a humidity sensor installed outside the target space SP, respectively.

In the present embodiment, as the intra-nap information D2, the sleep onset latency, the body motion, the heartbeat, the pulse, the respiration rate, and the LF/HF of the subject 90, and the indoor temperature, the indoor humidity, the illumination, and the noise of the target space SP are used.

### (2-1-5-1-3) Post-nap information

The acquisition part 191 acquires the post-nap information D3 regarding the subject 90 after the end of the nap of the subject 90. The post-nap information D3 includes at least one of a subjective evaluation of the subject 90 with respect to the level of satisfaction of the nap, a subjective evaluation of the subject 90 with respect to the thermal sensation during the nap, or a subjective evaluation of the subject 90 with respect to sleepiness after the nap.

In the present embodiment, as the post-nap information D3, the subjective evaluation for the sleepiness after the nap is used.

The acquisition part 191 acquires the post-nap information D3 from the subject 90 via the input unit 12 after the end of the nap of the subject 90.

### (2-1-5-2) Prediction part

The prediction part 192 predicts a cycle of sleepiness of the subject 90 from the pre-nap information D11. The cycle of sleepiness is information about a time zone in which the subject 90 feels sleepiness and a time zone in which the subject 90 hardly feels sleepiness. The cycle of sleepiness is related to, for example, a circadian rhythm of the subject 90. The circadian rhythm is, for example, information indicating that a person generally has the highest core body temperature around 4 p.m., and the lowest core body temperature around 4 a.m. Persons tend to feel strongly sleepiness in a time zone in which the core body temperature is low. During the daytime, persons generally tend to feel sleepiness between 2 p.m. and 4 p.m. due to the influence of a circasemidian rhythm. The circadian rhythm of the subject 90 is, for example, information about an average wake-up time and an average bed time of the subject 90. In addition, the circadian rhythm of the subject 90 is, for example, information indicating whether the subject 90 is of a morning type or an evening type. For example, it may be considered that, during the daytime, the subject 90 of the morning type feels sleepiness in an earlier time zone compared with general persons. When the wake-up time on the day of the nap and the bed time corresponding to the wake-up time on the day of the nap deviate from the average wake-up time and the average bed time of the subject 90, the time zone in which the subject 90 feels sleepiness may also deviate. Further, when the wake-up time on the day of the nap of the subject 90 is earlier than the average wake-up time, when the bed time corresponding to the wake-up time on the day of the nap of the subject 90 is later than the average bed time, or when the sleep time corresponding to the wake-up time on the day of the nap of the subject 90 is shorter than the average sleep time, the time zone in which the subject 90 feels sleepiness may be brought forward. In the present embodiment, the prediction part 192 predicts the cycle of sleepiness of the subject 90 from the wake-up time on the day of the nap, the bed time corresponding to the wake-up time on the day of the nap, the sleep time corresponding to the wake-up time on the day of the nap, the average wake-up time, the average bed time, the average sleep time, the evaluation value of the daily sleeping state, the subjective evaluation for sleepiness before the nap, the gender, and the age of the subject 90 which are the pre-nap information D11.

An administrator of the environmental control content determination device 1 constructs in advance a deep learning model for predicting the cycle of sleepiness from the pre-nap information D11, using the pre-nap information D11 of various persons and data associated with the cycle of sleepiness, for example. The prediction part 192 predicts the cycle of sleepiness by inputting the pre-nap information D11 to the deep learning model.

### (2-1-5-3) Determination part

The determination part 193 judges the wakefulness level of the subject 90 in the expected nap time zone D12 and determines the content of the environmental control for the target space SP in which the subject 90 takes a nap, based on a temporal relationship between the expected nap time zone D12 and the cycle of sleepiness.

Specifically, the determination part 193 specifies, from the cycle of sleepiness, a first time zone in which the subject feels sleepiness and is likely to sleep for a short time and a second time zone in which the subject is less likely to feel sleepiness and is likely to sleep for a short time. The first time zone is, for example, the afternoon or a time zone in which the core body temperature tends to decrease. The second time zone is, for example, in the morning or a time zone in which the core body temperature tends to rise. Then, the determination part 193 determines the control pattern depending on whether the expected nap time zone D12 corresponds to either of the first time zone and the second time zone. At this time, for example, when the sleep time corresponding to the wake-up time on the day of the nap is shorter than the average sleep time, when the wake-up time on the day of the nap is earlier than the average wake-up time, or when the bed time corresponding to the wake-up time on the day of the nap is later than the average bed time, it may be regarded that the expected nap time zone D12 corresponds to the first time zone even when corresponding to the second time zone.

In a case where the subject 90 has taken alcohol or caffeine such as coffee before taking a nap, the subject 90 is likely to wake up due to the alcohol or caffeine, and thus the determination part 193 may judge the wakefulness level of the subject 90 in consideration of the time and amount of alcohol or caffeine which has been taken. Further, in a case where a so-called "social jet lag", which is a difference between a middle time of the bedtime on a weekday and a middle time of the bedtime on a holyday, is for example, one hour or more, the determination part 193 may judge the wakefulness level of the subject 90 in consideration of the fact that the sleepiness is generally likely to occur. In addition, when the subject 90 is a female, the basal body temperature changes due to the influence of female hormones according to a menstrual cycle, and thus the determination part 193 may judge the wakefulness level of the subject 90 in consideration of the fact that the basal body temperature is low and the subject 90 is likely to feel sleepy during menstruation. On the other hand, the determination part 193 may judge the wakefulness level of the subject 90 in consideration of the fact that the basal body temperature rises and the subject is less likely to feel sleepy, on the contrary, after ovulation.

The determination part 193 determines the content of the environmental control by selecting from a plurality of control patterns. The control pattern is expressed by a form of a transition in a value of a control parameter related to the environment of the target space SP. In other words, the content of the environmental control includes a control pattern and a control parameter. The control parameter includes at least one of: an air volume, a wind direction, a wind speed, and an operating mode of the air conditioner 2 installed in the target space SP; the indoor temperature, the indoor humidity, the illumination, the sound, or the fragrance in the target space SP; or the inclination of the bedding. For example, when the control parameter is the indoor temperature of the target space SP, the control pattern means when to raise, keep constant, or lower the indoor temperature, but does not mean a change width of the indoor temperature (how much to raise or lower the indoor temperature). In the present embodiment, the determination part 193 determines the content of the environmental control by selecting from a first control pattern and a second control pattern. However, the present invention is not limited thereto, and the determination part 193 may determine the content of the environmental control by selecting from three or more control patterns. In the present embodiment, as the control parameter, the indoor temperature of the target space SP is used.

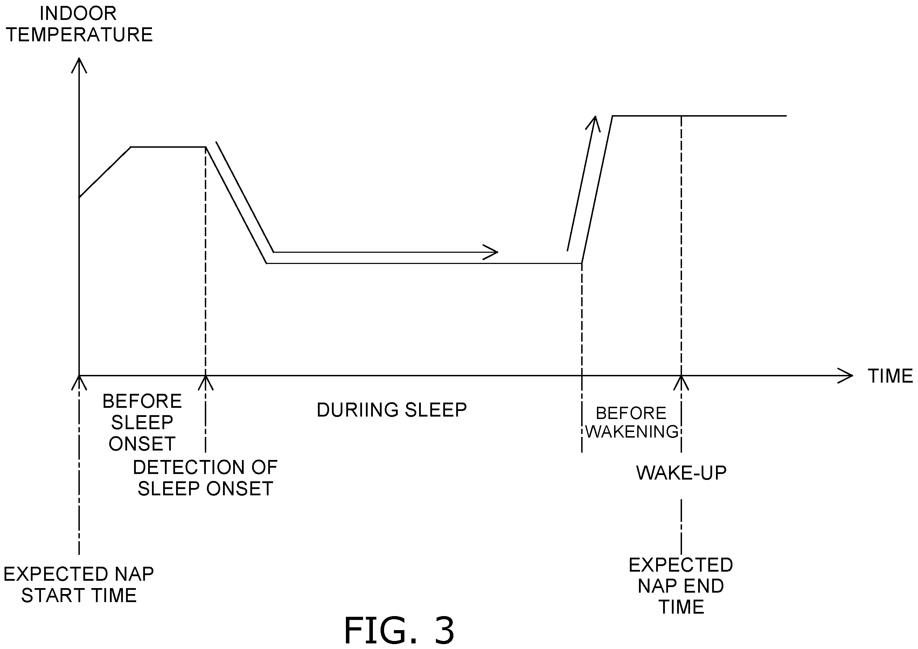

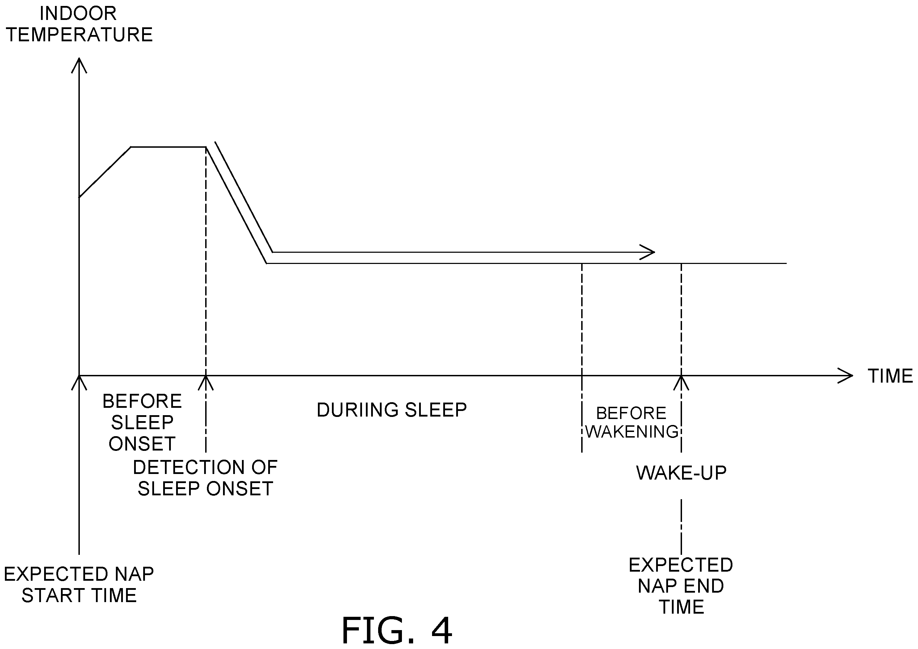

Fig. 3 is a diagram showing the first control pattern. Fig. 4 is a diagram showing the second control pattern. Figs. 3 and 4 show a form of a transition in the value of the indoor temperature of the target space SP until the expected nap end time from the expected nap start time (in other words, in the expected nap time zone D12).

When the expected nap time zone D12 corresponds to the first time zone, the determination part 193 judges that the subject 90 is in a state of being likely to enter deep sleep, and selects the first control pattern. As shown in Fig. 3, the first control pattern is a control to raise the indoor temperature before a sleep onset of the subject 90, to lower and maintain the indoor temperature after detection of the sleep onset, and to raise the indoor temperature at a predetermined time before the expected nap end time (hereinafter, may be referred to as a time zone before wakening). The predetermined time is, for example, 5 minutes. When the expected nap time zone D12 corresponds to the first time zone, since the subject 90 falls asleep too deeply when the indoor temperature during sleeping is maintained in the time zone before wakening, the determination part 193 selects the first control pattern in which the indoor temperature is raised before wakening. The environmental control content determination device 1 acquires a timing of sleep onset detection from, for example, the sleep meter attached to the bedding, and notifies the air conditioner 2.

When the expected nap time zone D12 corresponds to the second time zone, the determination part 193 judges that the subject 90 is in a state of being difficult to enter deep sleep, and selects the second control pattern. As shown in Fig. 4, the second control pattern is a control to raise the indoor temperature before a sleep onset of the subject 90 and to lower and maintain the indoor temperature after detection of the sleep onset. When the expected nap time zone D12 corresponds to the second time zone, since there is a high possibility that the subject 90 gets up on the way without taking sufficient sleep when the indoor temperature is raised in the time zone before wakening, the determination part 193 selects the second control pattern to maintain the indoor temperature during the sleep in the time zone before wakening.

The form of the transition in the value of the indoor temperature (control parameter) in the first control pattern and the second control pattern is determined for each subject 90. Regarding the subject 90 who uses the environmental control content determination device 1 for the first time, the form of the transition in the value of the indoor temperature (control parameter) in the first control pattern and the second control pattern may be determined to be a form of a transition in a value of the indoor temperature of a person who has the pre-nap information D11 similar to that of the subject 90 and is different from the subject 90, or a form of a transition in a value of the indoor temperature of a past person, who has determined by the update part 194 that a sufficient nap has been taken. Here, that the pre-nap information D11 is similar means that, for example, at least one of the information related to sleep, the information related to meals, the gender, the age, the generation, or the thermal sensation of the subject 90 in the pre-nap information D11 is the same. For example, a person who has the same thermal sensation of being hot or cold as the subject 90 is considered to be similar to the subject 90. Further, for example, a person who substantially has the average wake-up time or the average bed time the same as that of the subject 90 is considered to be similar to the subject 90. Further, regarding the subject 90 who uses the environmental control content determination device 1 for the first time, the form of the transition in the value of the indoor temperature in the first control pattern and the second control pattern may be determined to be a form of a transition in a value of a predetermined general-purpose indoor temperature.

Regarding the subject 90 who uses the environmental control content determination device 1 for the second and subsequent times, the form of the transition in the value of the indoor temperature in the first control pattern and the second control pattern is determined to be a form of a transition in a value of the previous indoor temperature or a form of a transition in a value of the indoor temperature previously updated by the update part 194.

### (2-1-5-4) Update part

The update part 194 updates the form of the transition in the value of the control parameter in the selected control pattern when it is determined, based on the pre-nap information D11, the intra-nap information D2, or the post-nap information D3 after the end of the nap of the subject 90, that the subject 90 did not take a sufficient nap or the subject 90 felt an unpleasant thermal sensation during the nap. For example, when the control parameter is the indoor temperature of the target space SP, the update part 194 updates the transition width (how much to raise or lower the indoor temperature) of the indoor temperature in the selected control pattern.

Specifically, the update part 194 determines that the subject has not taken a sufficient nap when the nap time is shorter than a threshold, when a time of a pred etermined sleep stage is shorter than a threshold, when a standard deviation of the heartbeat, the respiration rate, the pulse, or the LF/HF during sleep in the intra-nap information D2 is larger than a threshold, or when the subjective evaluation (post-nap information D3) for sleepiness after the nap is not better than the subjective evaluation (pre-nap information D11) for sleepiness before the nap. The nap time refers to, for example, the sum of the time of REM sleep and the time of non-REM sleep in the intra-nap information D2. The threshold of the nap time is, for example, a time corresponding to 70% of the expected nap time. The time of the predetermined sleep stage is, for example, a time of a second stage of non-REM sleep. The threshold of the time of the predetermined sleep stage is, for example, a time corresponding to 50% of the nap time. The update part 194 may determine that the subject has not taken a sufficient nap when the time during which a stable heartbeat can be maintained during the nap is shorter than the threshold, in the heartbeat of the subject 90 in the intra-nap information D2, for example.

When it is determined that the subject 90 has not taken a sufficient nap or the subject 90 feels an unpleasant thermal sensation during the nap in the first control pattern, the update part 194 reduces a gradient of the indoor temperature in the time zone before wakening in the first control pattern, for example. When it is determined that the subject 90 has not taken a sufficient nap or the subject 90 feels an unpleasant thermal sensation during the nap in the second control pattern, the update part 194 adjusts the indoor temperature during sleep in the second control pattern to an appropriate temperature, for example. Further, for example, when it is determined, from the subjective evaluation of the subject 90 for the thermal sensation during the nap, that the subject 90 feels an unpleasant thermal sensation during the nap due to the evaluation as being hot during the nap, the update part 194 lowers the indoor temperature during the nap in the first control pattern and the second control pattern. Further, for example, when it is determined, from the subjective evaluation of the subject 90 for the thermal sensation during the nap, that the subject 90 feels an unpleasant thermal sensation during the nap due to the evaluation as being cold during the nap, the update part 194 raises the indoor temperature during the nap in the first control pattern and the second control pattern. Further, for example, when it is determined, from the fact that the time of non-REM sleep is shorter than the threshold, that the subject 90 has not taken a sufficient nap, the update part 194 lowers the indoor temperature during the sleep in the first control pattern and the second control pattern. Further, for example, when it is determined, from the subjective evaluation of the subject 90 for the sleepiness after the nap, that the subject 90 feels an unpleasant thermal sensation during the nap due to the evaluation as being cold in the time zone before the sleep onset, the update part 194 raises (for example, by 1°C) the indoor temperature in the time zone before the sleep onset in the first control pattern and the second control pattern. Further, for example, when it is determined, from the subjective evaluation of the subject 90 for the sleepiness after the nap, that the subject 90 feels an unpleasant thermal sensation during the nap due to the evaluation as being hot in the time zone before the sleep onset, the update part 194 lowers (for example, by 1°C) the indoor temperature in the time zone before the sleep onset in the first control pattern and the second control pattern. Further, for example, when it is determined, from the subjective evaluation of the subject 90 for the level of satisfaction of the nap, that the subject 90 has not taken a sufficient nap due to "the evaluation as not being cold but not getting enough sleep during the nap", the update part 194 lowers (for example, by 1°C) the indoor temperature during the nap in the first control pattern and the second control pattern.

### (2-1-5-5) Transmission part

The transmission part 195 transmits the first control pattern or the second control pattern determined by the determination part 193 to the air conditioner 2.

### (2-2) Air conditioner

The air conditioner 2 constitutes a vapor compression refrigeration cycle and performs air conditioning of the target space SP. The air conditioner 2 mainly includes an indoor unit 20, an outdoor unit, and a controller. The indoor unit 20 and the outdoor unit constitutes a refrigerant circuit. Further, the indoor unit 20 and the outdoor unit are communicably connected to each other via a communication line.

In the present embodiment, as shown in Fig. 1, the indoor unit 20 is a wall-mounted unit installed on a wall of the target space SP. The indoor unit 20 mainly includes an indoor heat exchanger, an indoor fan, and an indoor control unit. The indoor heat exchanger performs heat exchange between refrigerant flowing through the indoor heat exchanger and air in the target space SP. The indoor fan is a fan that supplies air in the target space SP to the indoor heat exchanger. The indoor control unit controls operation of each of the units constituting the indoor unit 20. The indoor control unit includes a control computation device and a storage device.

The outdoor unit is installed outside the target space SP. The outdoor unit mainly includes a compressor, a flow path switching valve, an outdoor heat exchanger, an outdoor expansion valve, an outdoor fan, and an outdoor control unit. The compressor sucks low-pressure refrigerant, compresses the refrigerant using a compression mechanism, and discharges the compressed refrigerant, thereby circulating the refrigerant existing in the refrigerant circuit. The flow path switching valve is a mechanism that switches the flow path of the refrigerant between various operating modes including a cooling operation and a heating operation. The outdoor heat exchanger performs heat exchange between the refrigerant flowing through the outdoor heat exchanger and the air outside the target space SP. The outdoor expansion valve is a mechanism that adjusting pressure and flow rate of the refrigerant flowing through the refrigerant circuit. The outdoor fan is a fan that supplies the air outside target space SP to the outdoor heat exchanger. The outdoor control unit controls operations of each of the units constituting the outdoor unit. The outdoor control unit includes a control computation device and a storage device.

A controller includes the indoor control unit and the outdoor control unit. The controller controls the operations of the entire air conditioner 2 by causing the control computation devices of the indoor control unit and the outdoor control unit to execute programs stored in the storage devices, respectively. The controller controls an operating mode, a set temperature, an air volume, and the like based on the content of the environmental control received from the environmental control content determination device 1, and performs air conditioning of the target space SP. In the present embodiment, upon receiving the first control pattern or the second control pattern as the content of the environmental control from the environmental control content determination device 1, the air conditioner 2 performs air conditioning of the target space SP such that the target space SP reaches the indoor temperature indicated by the first control pattern or the second control pattern.

### (3) Processing

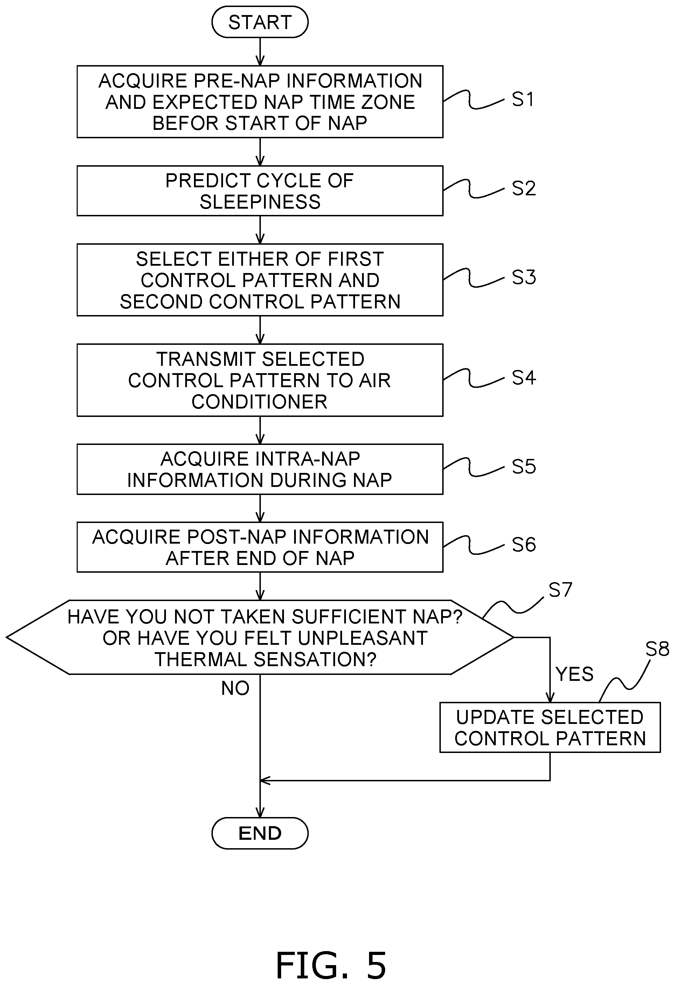

An example of processing of the environmental control content determination device 1 will be described with reference to a flowchart of Fig. 5.

As shown in step S1, the environmental control content determination device 1 acquires the pre-nap information D11 and the expected nap time zone D12 from the subject 90 via the input unit 12 before the subject 90 starts to nap.

When step S1 is finished, as shown in step S2, the environmental control content determination device 1 predicts the cycle of sleepiness of the subject 90 from the pre-nap information D11.

When step S2 is finished, as shown in step S3, the environmental control content determination device 1 selects one of the first control pattern and the second control pattern to determine the content of the environmental control. When the subject 90 uses the environmental control content determination device 1 for the first time, the form of the transition in the value of the indoor temperature in the first control pattern and the second control pattern is determined to be the form of the transition in the value of the indoor temperature of a person who has the pre-nap information D11 similar to that of the subject 90 and is different from the subject 90. When the subject 90 uses the environmental control content determination device 1 for the second or subsequent times, the form of the transition in the value of the indoor temperature in the first control pattern and the second control pattern is determined to be a form of a transition in a value of the previous indoor temperature or a form of a transition in a value of the indoor temperature previously updated by the update part 194.

When step S3 is finished, as shown in step S4, the environmental control content determination device 1 transmits the selected control pattern to the air conditioner 2. Upon receiving the control pattern, the air conditioner 2 performs air conditioning of the target space SP such that the target space SP reaches the indoor temperature indicated by the control pattern.

When step S4 is finished, as shown in step S5, the environmental control content determination device 1 acquires the intra-nap information D2 about the subject 90 during the nap of the subject 90.

When step S5 is finished, as shown in step S6, the environmental control content determination device 1 acquires the post-nap information D3 from the subject 90 via the input unit 12 after the end of the nap of the subject 90.

When step S6 is finished, as shown in step S7, the environmental control content determination device 1 determines, based on the pre-nap information D11, the intra-nap information D2, and the post-nap information D3, whether the subject 90 has not taken a sufficient nap or whether the subject 90 feels an unpleasant thermal sensation during the nap. When it is determined that the subject 90 has not taken a sufficient nap or the subject 90 feels an unpleasant thermal sensation during the nap, the process proceeds to step S8. When it is determined that the subject 90 has taken a sufficient nap or the subject 90 has not felt an unpleasant thermal sensation during the nap, the process ends.

When the process proceeds from step S7 to step S8, the environmental control content determination device 1 updates the form of the transition in the value of the indoor temperature in the selected control pattern, and ends the process.

### (4) Features

(4-1)
In order to provide a comfortable nap to a subject, conventionally, there is a technique of performing environmental control on a target space in which the subject takes a nap. However, the conventional technique has a problem that it is not sufficient for providing a comfortable nap to the subject.

The environmental control content determination device 1 of the present embodiment includes the acquisition part 191 and the determination part 193. The acquisition part 191 acquires, regarding the subject 90, the pre-nap information D11 and the expected nap time zone D12 including an expected nap start time and an expected nap end time. The determination part 193 judges a wakefulness level of the subject 90 in the expected nap time zone D12 based on the pre-nap information D11, and determines a content of environmental control for a target space SP in which the subject 90 takes a nap.

The environmental control content determination device 1 judges the wakefulness level of the subject 90 in the expected nap time zone D12 based on the pre-nap information D11, and determines the content of environmental control for the target space SP in which the subject 90 takes a nap. As a result, the environmental control content determination device 1 can provide the subject 90 with a more comfortable nap.

(4-2)
The environmental control content determination device 1 of the present embodiment further includes a prediction part 192. The prediction part 192 predicts a cycle of sleepiness of the subject 90 from the pre-nap information D11. The determination part 193 determines the content of environmental control based on a temporal relationship between the expected nap time zone D12 and the cycle of the sleepiness.

The cycle of sleepiness of the subject who takes a nap has not been conventionally taken into consideration, but the cycle of sleepiness has been now found to be an important factor for providing a comfortable nap to the subject 90. The environmental control content determination device 1 determines the content of environmental control for the target space SP in which the subject 90 takes a nap in consideration of the cycle of sleepiness of the subject 90 who takes a nap. As a result, the environmental control content determination device 1 can provide the subject 90 with a more comfortable nap.

(4-3)
In the environmental control content determination device 1 of the present embodiment, the pre-nap information D11 includes at least first information including information related to sleep of the subject 90.

(4-4)
In the environmental control content determination device 1 of the present embodiment, the information related to the sleep includes at least one of a wake-up time on the day of the nap, a bed time corresponding the wake-up time on the day of the nap, a sleep time corresponding the wake-up time on the day of the nap, an average wake-up time, an average bed time, an average sleep time, or an evaluation value of a daily sleeping state.

(4-5)
In the environmental control content determination device 1 of the present embodiment, the first information further includes at least one of information related to meals, a subjective evaluation for sleepiness before the nap, a subjective fatigue evaluation before the nap, a subjective stress evaluation before the nap, a behavior expectation after the nap, or a behavior history on the day of the nap.

(4-6)
In the environmental control content determination device 1 of the present embodiment, the information related to the meals includes at least one of: times when the subject 90 has a breakfast, a lunch, a snack, and a dinner; or meal contents of the breakfast, the lunch, the snack, and the dinner of the subject 90.

(4-7)
In the environmental control content determination device 1 of the present embodiment, the pre-nap information D11 further includes second information including at least one of a gender, an age, a generation, a clothing amount, or a thermal sensation of the subject 90.

(4-8)
In the environmental control content determination device 1 of the present embodiment, the determination part 193 determines the content of environmental control by selecting from a plurality of control patterns. The environmental control content determination device 1 can simplify the environmental control by selecting the content of the environmental control from a plurality of control patterns.

(4-9)
In the environmental control content determination device 1 of the present embodiment, the control pattern is expressed by a form of a transition in a value of a control parameter related to an environment of the target space SP.

(4-10)
In the environmental control content determination device 1 of the present embodiment, the control parameter includes at least one of: an air volume, a wind direction, a wind speed, and an operating mode of an air conditioner 2 installed in the target space SP; an indoor temperature, an indoor humidity, an illumination, a sound, or a fragrance in the target space SP; or an inclination of bedding.

(4-11)
In the environmental control content determination device 1 of the present embodiment, the control parameter is an indoor temperature of the target space SP. The control pattern includes a control to raise the indoor temperature before a sleep onset of the subject 90, to lower and maintain the indoor temperature after detection of the sleep onset, and to raise the indoor temperature at a predetermined time before the expected nap end time, and a control to raise the indoor temperature before a sleep onset of the subject 90 and to lower and maintain the indoor temperature after detection of the sleep onset.

(4-12)
In the environmental control content determination device 1 of the present embodiment, the determination part 193 specifies, from the cycle of sleepiness, a first time zone in which the sleepiness is felt and sleep is easily taken for a short time and a second time zone in which the sleepiness is hardly felt and the sleep is hardly taken for a short time. The determination part 193 selects the control pattern depending on whether the expected nap time zone D12 corresponds to either of the first time zone and the second time zone. As a result, the environmental control content determination device 1 can select the control pattern according to the cycle of sleepiness of the subject 90.

(4-13)
In the environmental control content determination device 1 of the present embodiment, the acquisition part 191 further acquires intra-nap information D2 regarding the subject 90 during the nap of the subject 90. The acquisition part 191 further acquires post-nap information D3 regarding the subject 90 after an end of the nap of the subject 90. The environmental control content determination device 1 further includes an update part 194. The update part 194 updates the form of the transition in the value of the control parameter. The update part 194 updates the form of the transition in the value of the control parameter, based on the pre-nap information D11, the intra-nap information D2, or the post-nap information D3, after the end of the nap of the subject 90. The environmental control content determination device 1 can provide a more comfortable nap at the time of the next nap by updating the form of the transition in the value of the control parameter.

(4-14)
In the environmental control content determination device 1 of the present embodiment, the intra-nap information D2 includes at least one of: a sleep depth, a heartbeat, a body motion, a pulse, a respiration rate, an LF/HF, a blood pressure, a body temperature, a sleep onset latency, and a brainwave of the subject 90; an indoor temperature, an indoor humidity, an airflow, an illumination, a sound, or a fragrance in the target space SP; a temperature of bedding; an inclination of the bedding; or an outdoor temperature or an outdoor humidity outside the target space SP.

(4-15)
In the environmental control content determination device 1 of the present embodiment, the post-nap information D3 includes at least one of a subjective evaluation of the subject 90 with respect to a level of satisfaction of the nap, a subjective evaluation of the subject 90 with respect to a thermal sensation during the nap, or a subjective evaluation of the subject 90 with respect to sleepiness after the nap.

(4-16)
In the environmental control content determination device 1 of the present embodiment, the form of the transition in the value of the control parameter for the subject 90 is determined to be: a form of a transition in a value of the control parameter of a person who has the pre-nap information D11 similar to that of the subject 90 and is different from the subject 90; or a form of a transition in a value of the control parameter of a past person, who has determined by the update part 194 that a sufficient nap has been taken.

(4-17)
In the environmental control content determination device 1 of the present embodiment, the pre-nap information D11 includes a subjective evaluation for sleepiness before a nap. The intra-nap information D2 includes at least one of a brainwave, a heartbeat, a pulse, an LF/HF, a sleep depth, a respiration rate, or a body temperature. The post-nap information D3 includes at least one of a subjective evaluation of the subject 90 with respect to the level of satisfaction of the nap, a subjective evaluation of the subject 90 with respect to the thermal sensation during the nap, or a subjective evaluation of the subject 90 with respect to sleepiness after the nap. The update part 194 updates the form of the transition in the value of the control parameter when it is determined that the subject 90 has not taken a sufficient nap or the subject 90 has felt an unpleasant thermal sensation during the nap.

(4-18)
In the environmental control content determination device 1 of the present embodiment, the update part 194 determines that the sufficient nap has not been taken when a nap time is shorter than a threshold, when a time of a predetermined sleep stage is shorter than a threshold, when a standard deviation of a heartbeat, a respiration rate, a pulse, or an LF/HF during sleep is larger than a threshold, or when a subjective evaluation for sleepiness after the nap is not better than a subjective evaluation for sleepiness before the nap.

(4-19)
The environmental control content determination method of the present embodiment includes an acquisition step S1 and a determination step S3. The acquisition step S1 includes acquiring pre-nap information D11 and an expected nap time zone D12 including an expected nap start time and an expected nap end time regarding a subject 90. The determination step S3 includes determining, based on the pre-nap information D11, a content of environmental control for a target space SP in which the subject 90 takes a nap.

(4-20)
The environmental control content determination method of the present embodiment further includes a prediction step S2. The prediction step S2 includes predicting a cycle of sleepiness of the subject 90 from the pre-nap information D11. The determination step S3 includes determining the content of environmental control based on a temporal relationship between the expected nap time zone D12 and the cycle of the sleepiness.

### (5) Modifications

### (5-1) Modification 1A

In the present embodiment, the pre-nap information D11 includes the first information and the second information. However, the pre-nap information D11 may further include at least one of an eyelid opening/closing state, an eyelid motion, a respiration rate, a pulse, a blood pressure, an LF/HF, a brainwave, a body temperature, or an in-vivo blood glucose level of the subject 90.

### (5-2) Modification 1B

In the present embodiment, the determination part 193 selects the control pattern depending on whether the expected nap time zone D12 corresponds to either of the first time zone and the second time zone. However, the determination part 193 may further select the control pattern, based on whether a sleep time corresponding to a wake-up time on the day of the nap is shorter than a predetermined time with respect to an average sleep time of the subj ect 90, or whether an evaluation value of a daily sleeping state of the subject 90 exceeds a threshold.

### (5-3) Modification 1C

In the present embodiment, the subject 90 directly inputs various kinds of information such as the pre-nap information D11 to the environmental control content determination device 1 via the input unit 12. However, the environmental control content determination device 1 may be installed on a cloud. In this case, the subject 90 inputs various kinds of information to the environmental control content determination device 1 from a user terminal such as a tablet via a network NW such as Internet.

In addition, the controller of the air conditioner 2 may have the function of the environmental control content determination device 1. In this case, the subject 90 inputs various kinds of information to the controller of the air conditioner 2 from the user terminal such as a tablet via a network NW such as a wireless LAN.

### (5-4) Modification 1D

In the present embodiment, the control parameter is the indoor temperature of the target space SP. However, the control parameter may be an illumination, a sound, or a fragrance in the target space SP, or an inclination of the bedding.

When the control parameter is the illumination, for example, control is performed to increase the illumination in response to the control for raising the indoor temperature. Further, when the control parameter is the sound, for example, control is performed to emit a loud sound in response to the control for raising the indoor temperature. Further, when the control parameter is the fragrance, for example, control is performed to generate a large amount of fragrance in response to the control for raising the indoor temperature. Further, when the control parameter is the inclination of the bedding, for example, control is performed to set the inclination of the bedding to a steep angle in response to the control for raising the indoor temperature.

(5-5)
While the embodiment of the present disclosure has been described above, it will be understood that various changes in form and detail may be made therein without departing from the spirit and scope of the present disclosure as set forth in the appended claims.

### Reference Signs List

1 ENVIRONMENTAL CONTROL CONTENT DETERMINATION DEVICE
191 ACQUISITION PART
192 PREDICTION PART
193 DETERMINATION PART
194 UPDATE PART
2 AIR CONDITIONER
90 SUBJECT
D11 PRE-NAP INFORMATION
D12 EXPECTED NAP TIME ZONE
D2 INTRA-NAP INFORMATION
D3 NAP END INFORMATION
S1 ACQUISITION STEP
S2 PREDICTION STEP
S3 DETERMINATION STEP
SP TARGET SPACE

### Citation List

### Patent Literature

PTL 1: Japanese Patent No. 6775949

## Claims

1. An environmental control content determination device (1) comprising: an acquisition part (191) that acquires pre-nap information (D11) and an expected nap time zone (D12) including an expected nap start time and an expected nap end time regarding a subject (90); and
a determination part (193) that judges a wakefulness level of the subject in the expected nap time zone based on the pre-nap information, and determines a content of environmental control for a target space (SP) in which the subject takes a nap.

2. The environmental control content determination device (1) according to claim 1, further comprising a prediction part (192) that predicts a cycle of sleepiness of the subject from the pre-nap information, wherein
the determination part determines the content of environmental control based on a temporal relationship between the expected nap time zone and the cycle of the sleepiness.

3. The environmental control content determination device (1) according to claim 1 or 2, wherein the pre-nap information includes at least first information including information related to sleep of the subject.

4. The environmental control content determination device (1) according to claim 3, wherein the information related to the sleep includes at least one of a wake-up time on the day of the nap, a bed time corresponding the wake-up time on the day of the nap, a sleep time corresponding the wake-up time on the day of the nap, an average wake-up time, an average bed time, an average sleep time, or an evaluation value of a daily sleeping state.

5. The environmental control content determination device (1) according to claim 3 or 4, wherein the first information further includes at least one of information related to meals, a subjective evaluation for sleepiness before the nap, a subjective fatigue evaluation before the nap, a subjective stress evaluation before the nap, a behavior expectation after the nap, or a behavior history on the day of the nap.

6. The environmental control content determination device (1) according to claim 5, wherein the information related to meals includes at least one of:
times when the subject has a breakfast, a lunch, a snack, and a dinner; or
meal contents of the breakfast, the lunch, the snack, and the dinner of the subject.

7. The environmental control content determination device (1) according to any one of claims 3 to 6, wherein the pre-nap information further includes second information including at least one of a gender, an age, a generation, a clothing amount, or a thermal sensation of the subj ect.

8. The environmental control content determination device (1) according to any one of claims 3 to 7, wherein the pre-nap information further includes at least one of an eyelid opening/closing state, an eyelid motion, a respiration rate, a pulse, a blood pressure, an LF/HF, a brainwave, a body temperature, or an in-vivo blood glucose level of the subject.

9. The environmental control content determination device (1) according to any one of claims 1 to 8, wherein the determination part determines the content of environmental control by selecting from a plurality of control patterns.

10. The environmental control content determination device (1) according to claim 9, wherein the control pattern is expressed by a form of a transition in a value of a control parameter related to an environment of the target space.

11. The environmental control content determination device (1) according to claim 10, wherein the control parameter includes at least one of: an air volume, a wind direction, a wind speed, or an operating mode of an air conditioner (2) installed in the target space; an indoor temperature, an indoor humidity, an illumination, a sound, or a fragrance in the target space; or an inclination of bedding.

12. The environmental control content determination device (1) according to claim 10 or 11, wherein the control parameter is an indoor temperature of the target space, and
the control pattern includes
a control to raise the indoor temperature before a sleep onset of the subject, to lower and maintain the indoor temperature after detection of the sleep onset, and to raise the indoor temperature at a predetermined time before the expected nap end time, and
a control to raise the indoor temperature before a sleep onset of the subject and to lower and maintain the indoor temperature after detection of the sleep onset.

13. The environmental control content determination device (1) according to any one of claims 9 to 12, wherein
the determination part specifies, from the cycle of sleepiness, a first time zone in which the sleepiness is felt and sleep is easily taken for a short time and a second time zone in which the sleepiness is hardly felt and the sleep is hardly taken for a short time, and
the determination part selects the control pattern depending on whether the expected nap time zone corresponds to either of the first time zone and the second time zone.

14. The environmental control content determination device (1) according to claim 13, wherein
the determination part further selects the control pattern, based on whether a sleep time corresponding to a wake-up time on the day of the nap is shorter than a predetermined time with respect to an average sleep time of the subject, or
whether an evaluation value of a daily sleeping state of the subject exceeds a threshold.

15. The environmental control content determination device (1) according to any one of claims 10 to 14, wherein the acquisition part further acquires
intra-nap information (D2) regarding the subject during the nap of the subject, and
post-nap information (D3) regarding the subject after an end of the nap of the subject,
the environmental control content determination device further comprising an update part (194) that updates the form of the transition in the value of the control parameter, wherein
the update part updating the form of the transition in the value of the control parameter, based on the pre-nap information, the intra-nap information, or the post-nap information, after the end of the nap of the subject.

16. The environmental control content determination device (1) according to claim 15, wherein the intra-nap information includes at least one of: a sleep depth, a heartbeat, a body motion, a pulse, a respiration rate, an LF/HF, a blood pressure, a body temperature, a sleep onset latency, or a brainwave of the subject; an indoor temperature, an indoor humidity, an airflow, an illumination, a sound, or a fragrance in the target space; a temperature of bedding; an inclination of the bedding; or an outdoor temperature or an outdoor humidity outside the target space.

17. The environmental control content determination device (1) according to claim 15 or 16, wherein the post-nap information includes at least one of a subjective evaluation of the subject with respect to a level of satisfaction of the nap, a subjective evaluation of the subject with respect to a thermal sensation during the nap, or a subjective evaluation of the subject with respect to sleepiness after the nap.

18. The environmental control content determination device (1) according to any one of claims 15 to 17, wherein the form of the transition in the value of the control parameter for the subject is determined to be:
a form of a transition in a value of the control parameter of a person who has the pre-nap information similar to that of the subject and is different from the subject; or
a form of a transition in a value of the control parameter of a past person, who has determined by the update part that a sufficient nap has been taken.

19. The environmental control content determination device (1) according to any one of claims 15 to 18, wherein the pre-nap information includes a subjective evaluation for sleepiness before the nap,
the intra-nap information includes at least one of a brainwave, a heartbeat, a pulse, an LF/HF, a sleep depth, a respiration rate, or a body temperature,
the post-nap information includes at least one of a subjective evaluation of the subject with respect to a level of satisfaction of the nap, a subjective evaluation of the subject with respect to a thermal sensation during the nap, or a subjective evaluation of the subject with respect to sleepiness after the nap, and
the update part updates the form of the transition in the value of the control parameter when it is determined that the subject has not taken a sufficient nap or the subject has felt an unpleasant thermal sensation during the nap.

20. The environmental control content determination device (1) according to claim 18 or 19, wherein the update part determines that the sufficient nap has not been taken when a nap time is shorter than a threshold, when a time of a predetermined sleep stage is shorter than a threshold, when a standard deviation of a heartbeat, a respiration rate, a pulse, or an LF/HF during sleep is larger than a threshold, or when a subjective evaluation for sleepiness after the nap is not better than a subjective evaluation for sleepiness before the nap.

21. An environmental control content determination method comprising: an acquisition step (S1) of acquiring pre-nap information (D11) and an expected nap time zone (D12) including an expected nap start time and an expected nap end time regarding a subject (90); and
a determination step (S3) of determining, based on the pre-nap information, a content of environmental control for a target space (SP) in which the subject takes a nap.

22. The environmental control content determination method according to claim 21, further comprising a prediction step (S2) of predicting a cycle of sleepiness of the subject from the pre-nap information, wherein
the determination step including determining the content of environmental control based on a temporal relationship between the expected nap time zone and the cycle of the sleepiness.
